# EUROPEAN PATENT APPLICATION

(11) **EP 1 688 092 A2**
(43) Date of publication of application: **09.08.2006**
(21) Application number: 06002151.6
(22) Date of filing: 02.02.2006
(51) Int. Cl.: A61B 6/00, G01T 1/00

(54) **Radiological inspection apparatus and radiological inspection method**

(30) Priority: 04.02.2005 JP 2005029176
(71) Applicant: HITACHI, LTD., Chiyoda-ku Tokyo 100-8280 (JP)
(72) Inventor: Kojima, Shinichi c/o Hitachi, Ltd. I. P. Office, Chiyoda-ku, Tokyo 100-8220 (JP); Ueno, Yuuichirou c/o Hitachi, Ltd. I. P. Office, Chiyoda-ku, Tokyo 100-8220 (JP); Kobashi, Keiji c/o Hitachi, Ltd. I. P. Office, Chiyoda-ku, Tokyo 100-8220 (JP); Ishitsu, Takafumi c/o Hitachi, Ltd. I. P. Office, Chiyoda-ku, Tokyo 100-8220 (JP); Amemiya, Kensuke c/o Hitachi, Ltd. I. P. Office, Chiyoda-ku, Tokyo 100-8220 (JP)
(74) Representative: Strehl Schübel-Hopf & Partner

(57) **Abstract**

In a radiological inspection apparatus, a subject (17) lying on a bed (16) is located in the center of an imaging device (2). A large number of radiological detectors (4) are arranged around the subject 17. The radiological detectors (4) are arranged in multiple stages in a direction normal to the body axis of the subject (17). The radiological detectors (4a, 4b, and 4c) are sequentially arranged in three stages in a direction away from the subject (17). A large number of combinations each of the three radiological detectors 4 are installed like a ring so as to surround the subject 17. If circuits in the radiological detectors (4) detect a plurality of signals considered to be coincident, the system selects one of the radiological detectors (4) which is closer to the subject (7) depending on the energy of radiation.

## Description

### BACKGROUND OF THE INVENTION

The present invention relates to a radiological inspection apparatus including a medical radiological apparatus, and a radiological inspection method for improving the detection sensitivity of the radiological inspection apparatus using a simple method.

Techniques for imaging the functions of the body in an unaggressive manner include PET (Positron Emission Tomography) and SPECT (Single Photon Emission Computed Tomography) inspections.

The PET inspection is a method of adding a positron emission nuclide to pharmaceuticals, administering the pharmaceuticals to a subject to check what sites of the body consumes more of the pharmaceuticals. For example, whether or not the subject has a cancer can be determined by administering fluorodeoxyglucol to the subject as PET pharmaceuticals and examining the object; fluorodeoxyglucol is a kind of sugar that tends to accumulate in a cancerous site. Positrons emanated by the PET pharmaceuticals couple to nearby electrons to cause positron annihilation, thus emitting a pair of gamma rays (hereinafter, referred to as γ rays) having 511-keV energy. The γ rays are emanated in the opposite directions. Accordingly, the two detection elements between which the positrons have been emanated can be determined by using y ray detectors to detect the γ ray pair. Detecting a large number of the γ ray pairs enables the determination of sites that consume more of the pharmaceuticals. For example, using sugar as pharmaceuticals enables discovery of a cancerous site involved in intense sugar metabolism. In this case, data obtained indicates the number of γ ray pairs generated between the detectors. Thus, the number must be converted into data on voxels (fine cubes constituting a three-dimensional image). An example of a method for the conversion is a filtered back projection method, described in IEEE Transactions on Nuclear Science, Vol. NS-21, 1974, p.21.

SPECT involves administering radiopharmaceuticals containing a single photon emission nuclide to the subject and using γ ray detectors to detect γ rays emanated by the nuclide. A γ ray emanated by a single photon emission nuclide often used for SPECT inspections has energy on the order of several 100 keV. With SPECT, a single γ ray is emanated, so that it is impossible to determine the angle at which the γ ray is incident on a detection element. Thus, the incident angle of the γ ray is fixed by using a collimator to detect only the γ ray incident at a predetermined angle. SPECT is an inspection method of administering, to the subject, radiopharmaceuticals containing a substance that accumulates in a particular tumor or molecule as well as a single photon emission nuclide (⁹⁹Tc, ⁶⁷Ga, ²⁰¹Tl, or the like) and detecting a γ ray generated by the radiopharmaceuticals to determine a site that consumes more of the agent. Also with SPECT, data obtained is converted into data on voxels using a method such as filtered back projection. SPECT is often used to acquire transmission data (data on measurements of γ ray transmittance of the body). Further, the half life of ⁹⁹Tc, ⁶⁷Ga, or ²⁰¹Tl, used for SPECT, is longer than that of a radio-isotope used for PET, about six hours to three days.

With the PET or SPECT inspection, γ rays scattered in the body of the testing object have their advancing directions varied during the scattering. Consequently, such γ rays detected cannot be utilized to identify a particular site of the body but result in mere noise. Thus, the loss of energy during scattering is applied to the conventional techniques; an energy threshold is provided so as to utilize only γ ray data equal to or exceeding the energy threshold.

However, γ rays may be scattered not only in the body but also in detectors. Conventional PET and SPECT apparatuses independently process γ rays scattered in a plurality of detectors. Consequently, if the total of energy attenuated by the detectors exceeds the energy threshold but not the energy detected by each detector, all the γ rays are considered to be scattered radiations. Conventionally, such data is not utilized.

Some conventional methods of using data attenuated by a plurality of detectors to improve detection efficiency use data detected by a plurality of detectors as shown in JP-A-2000-321357 (paragraphs 0045 to 0056 and Figs. 8 and 9) or JP-A-2003-255048 (paragraphs 0048 to 0049 and Fig. 9). An improvement in detection efficiency enables high-quality images to be obtained in a short time. This contributes to reducing the exposure of the subject to radiation. If a plurality of scattered γ rays are used for PET or SPECT, it is necessary to estimate an initial scattering position, that is, which of the plurality of detectors has initially detected the signal. In this case, in view of the operating frequencies and processing accuracies of a CPU and circuits, it is difficult to implement a method of comparing the times at which a plurality of scattered γ rays considered to be coincident were detected, to determine a detector having detected a γ ray earliest to correspond to the initial scattering position. This is because γ rays travel about 30 cm per nsec. and because, for example, a CPU having a duty cycle of about several nsec cannot provide a sufficient time resolution.

However, the method described in JP-A-2000-321357 disadvantageously requires a circuit that calculates the sum of energies and a circuit that finds a difference between energies to determine which energy is higher. Further, JP-A-2003-255048, utilizing pair data for PET, disadvantageously requires a complicated circuit because scattering angles are determined from the positions at which simultaneously emanated γ rays have been detected and information on a plurality of detector positions associated with the scattering. Such a complicated circuit is a factor increasing the costs of the circuit, the quantity of heat generated by the circuit, and the time required for processing.

In view of these problems, it is thus an object of the present invention to provide a radiological inspection apparatus having a simplified circuit that determines the initial incident position of radiation.

### SUMMARY OF THE INVENTION

To accomplish the above object, the present invention provides a radiological inspection apparatus comprising radiological detectors at least installed in a plurality of stages along a direction normal to a central axis of the radiological inspection apparatus to detect radiation from a testing object located in a center of the detectors or a vicinity of the center, wherein if a plurality of radiological detectors detect radiation, an order in which the radiation is scattered is estimated using a positional relationship between the plurality of radiological detectors and a central axis of the radiological inspection apparatus. In estimating the scattering order, one of the plurality of radiological detectors which is closest to the central axis of the radiological inspection apparatus may be estimated as an initial scattering position. Further, the radiological detectors may be arranged like a ring around the central axis of the radiological inspection apparatus. The present invention includes a radiological inspection method for the radiological inspection apparatus.

The present invention provides a radiological inspection apparatus having a simplified circuit that determines the initial incident position of radiation.

Other objects, features and advantages of the invention will become apparent from the following description of the embodiments of the invention taken in conjunction with the accompanying drawings.

### BRIEF DESCRIPTION OF THE DRAWINGS

Fig. 1 is a schematic sectional view showing the basic configuration of an imaging device in accordance with an embodiment of the present invention;
Fig. 2 is a graph showing the relationship between the scattering angle and scattering probability of a γ ray in accordance with the embodiment of the present invention;
Fig. 3 is a diagram showing the configuration of a radiological inspection apparatus in accordance with the embodiment of the present invention;
Fig. 4 is a diagram showing the arrangement of radiological detectors in accordance with the embodiment of the present invention;
Fig. 5 is a graph showing the distribution of scattering angles of 511-keV γ rays used for PET in accordance with the embodiment of the present invention;
Fig. 6 is a diagram showing a circuit configuration for analog processing in accordance with the embodiment of the present invention;
Fig. 7 is a diagram showing a data format used for digital processing in accordance with the embodiment of the present invention;
Fig. 8 is a diagram showing the configuration of a lookup table in accordance with the embodiment of the present invention; and
Fig. 9 is a flowchart showing processing for estimating a γ ray incident position in accordance with the embodiment of the present invention.

### DETAILED DESCRIPTION OF THE INVENTION

With reference to the drawings, a detailed description will be given of the best mode for carrying out the present invention.

### <Basic principle of the apparatus>

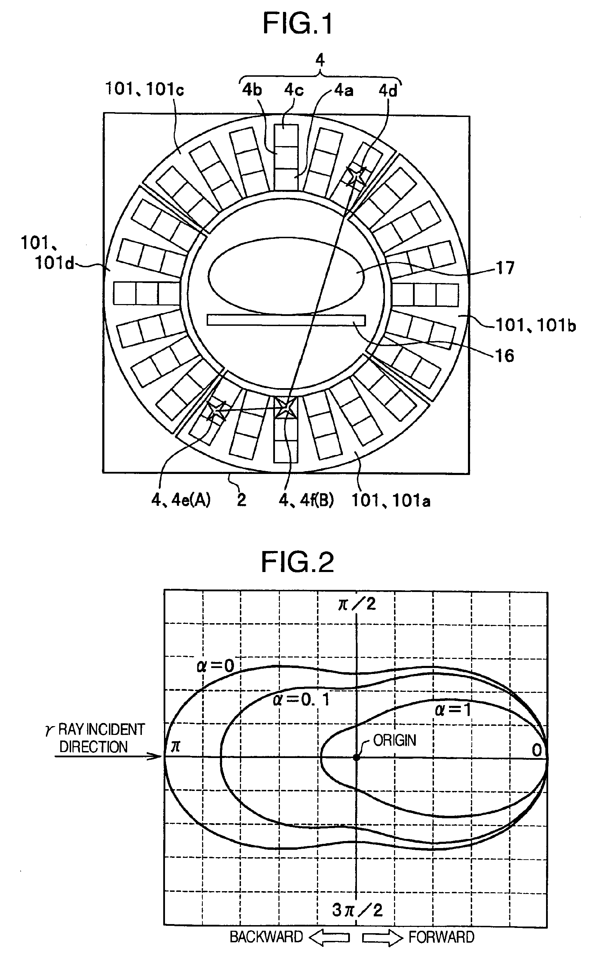

Fig. 1 is a schematic sectional view showing the basic configuration of an imaging device in accordance with an embodiment of the present invention. In Fig. 1, a subject (testing object) 17 lying on a bed 16 is located in the center of an imaging device 2. A large number of radiological detectors 4 (4a, 4b, 4c, 4d, 4e, 4f, ...) are arranged around the subject 17. The radiological detectors 4 are arranged in multiple stages in a direction normal to the body axis of the subject 17. In Fig. 1, the radiological detectors 4a, 4b, and 4c are sequentially stacked in three stages in a direction away from the subject 17. A large number of combinations each of three radiological detectors are installed like a ring so as to surround the subject 17. The large number of radiological detectors 4 arranged is divided into four units (101a, 101b, 101c, and 101d). Each of the four units 101 processes signals for γ rays detected by the radiological detectors 4.

A system uses the radiological detectors 4, shown in Fig. 1, to perform the following operation. If circuits in the radiological detectors 4 detect a plurality of signals, which are considered to be coincident, the system selects one of the detectors which is closer to or further from the subject depending on the energy of radiation. The system thus determines which γ ray was incident earlier. A detailed description will be given below.

Fig. 2 is a graph showing the relationship between the scattering angle and scattering probability of a γ ray. Specifically, the figure shows the scattering direction and scattering probability of a γ ray which is incident from left of the drawing and which is scattered at the position of origin of coordinates. α denotes the energy of an incident γ ray, indicating how many times as large as 1 m₀c² (= 511 keV) the energy is. For example, for α = 0.1, the incident energy is 51.1 keV. Further, a direction from the origin corresponds to a scattering direction, and the distance from the origin to a curve corresponds to scattering probability. Here, in the drawing, a direction extending rightward from the origin corresponds to a forward direction, whereas a direction extending leftward from the origin corresponds to a backward direction.

For α ≈ 0, that is, if the energy is sufficiently lower than 511 keV, the probability of forward scattering is equal to that of backward scattering. On the other hand, for α 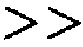 0, that is, if the energy is close to 511 keV, the probability of forward scattering is higher than that of backward scattering. Since the probability of forward scattering is thus higher, if a circuit capable of precisely acquiring signals for all the energies is made and used to detect γ rays, the radiological detector 4 closer to the central axis of the radiological inspection apparatus is likely to correspond to the initial scattering position.

### <Configuration of the apparatus>

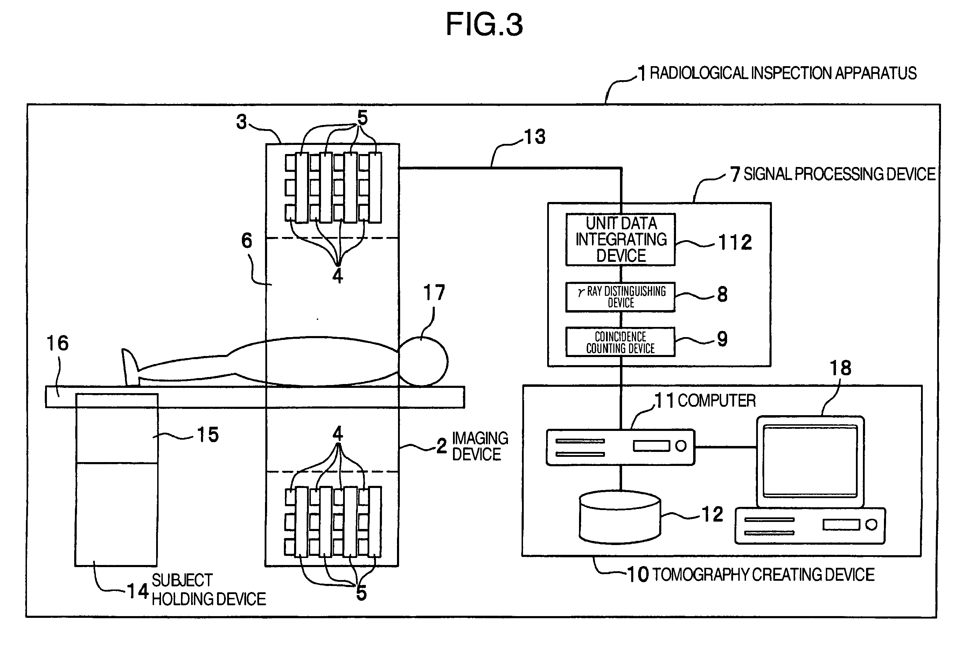

Fig. 3 is a diagram showing the configuration of a radiological inspection apparatus in accordance with the embodiment of the present invention. A radiological inspection apparatus 1 is used for PET inspections. The radiological inspection apparatus 1 comprises the imaging device 2, a signal processing device 7, a tomography creating device 10, and a subject holding device 14.

The imaging device 2 has a casing 3, a large number of radiological detectors 4, and a radiological detector support plate 5. The casing 3 has a through-hole 6 through which the subject 17, a testing object, is inserted. The large number of (for example, 10,000) radiological detectors 4 are arranged in the axial direction of the through-hole 6 so as to surround the through-hole 6. The innermost radiological detectors 4 are annularly arranged around the through-hole 6 as shown Fig. 1. The other radiological detectors 4 are arranged radially from the center of the through-hole 6 with respect to the innermost radiological detectors 4. That is, the radiological detectors 4 are also arranged at different positions along the radial direction of the through-hole 6. In the present embodiment, three radiological detectors 4 (for example, the radiological detectors 4a, 4b, and 4c shown in Fig. 1) are linearly arranged so as to constitute three stages along the radial direction of the through-hole 6. The radiological detectors 4 in each stage are concentrically arranged. The radiological detectors 4 need not be arranged in three stages but another number of radiological detectors may be arranged in the radial direction of the through-hole 6.

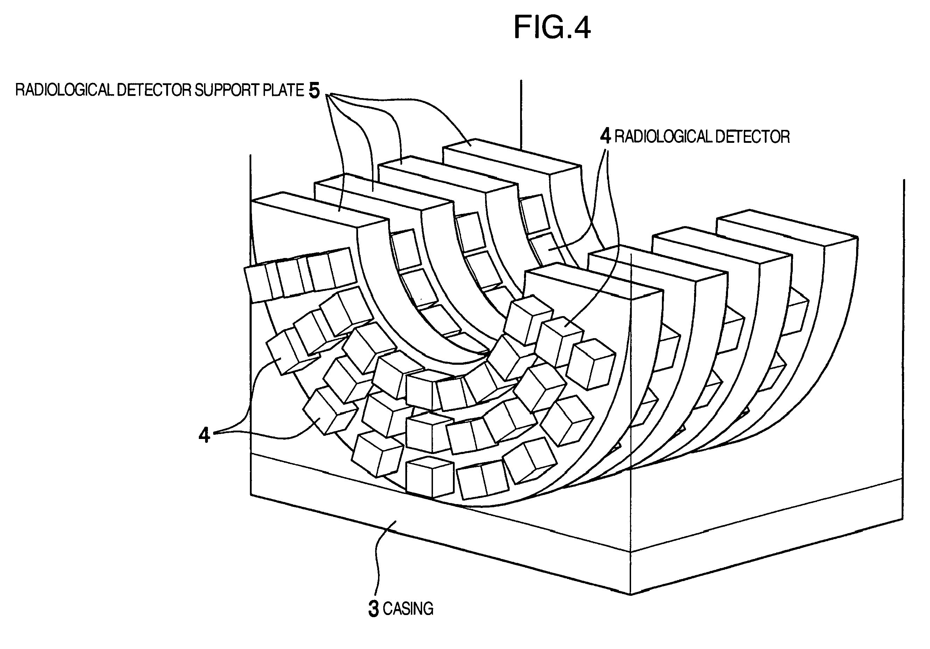

As shown in Fig. 4, the radiological detectors 4 are attached to a side surface of a radiological detector support plate 5. That is, the radiological detectors 4 are radially attached to the side surface of the radiological detector support plate 5, shaped like a half ring. A plurality of the radiological detector support plates 5 are arranged below the through-hole 6 in its axial direction; the radiological detectors 4 are attached to each of the radiological detector support plates 5. The radiological detector support plates 5 are fixed to the casing 3. Although not shown in Fig. 4, a plurality of the radiological detector support plates 5 are also arranged above the through-hole 6 in its axial direction; the radiological detectors 4 are attached to each of the radiological detector support plates 5. Each of the plurality of the radiological detector support plates 5 arranged below the through-hole 6 is placed to form a ring in the same plane together with a corresponding one of the plurality of the radiological detector support plates 5 arranged above the through-hole 6. The radiological detector support plate 5 may be annularly formed.

The radiological detector 4 is divided into four units 101a to 101d, as shown Fig. 1. Each unit 101 transmits data from all the radiological detectors 4 belonging to that unit. The unit 101 indicates the range of radiological detectors 4 effective in summing energy values if a γ ray scatters inside each radiological detector 4.

Referring back to Fig. 3, the signal processing device 7 comprises a unit data integrating device 112, a γ ray distinguishing device 8, and a coincidence counting device 9. The unit data integrating device 112 is provided for each of the units 101a to 101d, and has a function to integrate data for each unit. The unit data integrating device 112 is connected to the radiological detectors 4 through wires 13 provided for the respective radiological detectors 4. The γ ray distinguishing device 8 has a function for removing signals for scattered γ rays from input signals. The γ ray distinguishing device 8 is connected to the unit data integrating device 112. The coincidence counting device 9 has a function for determining whether or not two signal data have occurred simultaneously. The coincidence counting device 9 is connected to the γ ray distinguishing device 8.

The tomography creating device 10 comprises a computer 11, a storage device 12, and a display device 18. The computer 11 is connected to the coincidence counting device 9. The storage device 12 is connected to the computer 11. The display device 18 is connected to the computer 11.

The subject holding device 14 comprises a support member 15 and the bed 16 installed at the upper end of the support member 15 so as to be movable in the longitudinal direction of the support member 15. The imaging device 2 is placed in a direction orthogonal to the longitudinal direction of the bed 16.

Typical examples of the radiological detector 4 include a scintillator and a semiconductor radiological detector. The scintillator requires a photomultiplier or the like to be placed behind a crystal (BGO, NaI, or the like) serving as a radiation detecting section. The scintillator is thus unsuitable for the radiological detectors stacked in multiple stages, for example, three stages as previously described. The semiconductor photomultiplier does not require any photomultipliers or the like and is thus suitable for the stacked arrangement. In the present embodiment, the semiconductor radiological detector is used as the radiological detector 4. A 5-mm cubic constituting a detection section is composed of cadmium telluride (CdTe). The detection section may be composed of gallium arsenide (GaAs) or cadmium zinc telluride (CZT).

### <Processing by the apparatus>

Now, with reference to Fig. 3, description will be given of an inspection procedure using the radiological inspection apparatus.

First, PET radiopharmaceuticals are pre-administered into the body of the subject 17 through an injection or the like. A predetermined time is allowed to elapse until the radiopharmaceuticals diffuse within the body and gather in a diseased site so that the diseased site can be imaged. The radiopharmaceuticals are selected in accordance with the diseased site to be inspected. The elapse of the predetermined time causes the radiopharmaceuticals to gather in the diseased site (for example, a cancerous site) of the subject 17. After a predetermined time elapses, the subject 17 lies on the bed 16 of the subject holding device 14. Depending on the type of the inspection, the radiopharmaceuticals may be administered to the subject 17 lying on the bed 16. Alternatively, the radiopharmaceuticals may be administered to the subject 17 being imaged by the imaging device 2.
Alternatively, depending on the type of the radiological inspection apparatus 1, transmission data may be acquired.

Then, the bed 16 on which the subject 17 is lying is moved toward the imaging device 2. The subject 17 and the bed 16 are inserted into the through-hole 6 from the left of the drawing and moved toward the right of the drawing. γ rays of 511 keV emanated by the diseased site in the body of the subject 17 are incident on the radiological detectors 4.

Each of the radiological detectors 4 detects a γ ray emanated by the diseased site of the subject 17 in association with the radiopharmaceuticals. The radiological detector 4 then outputs a signal containing a γ ray detection signal and sends it to the unit data integrating device 12 through the wire 13.

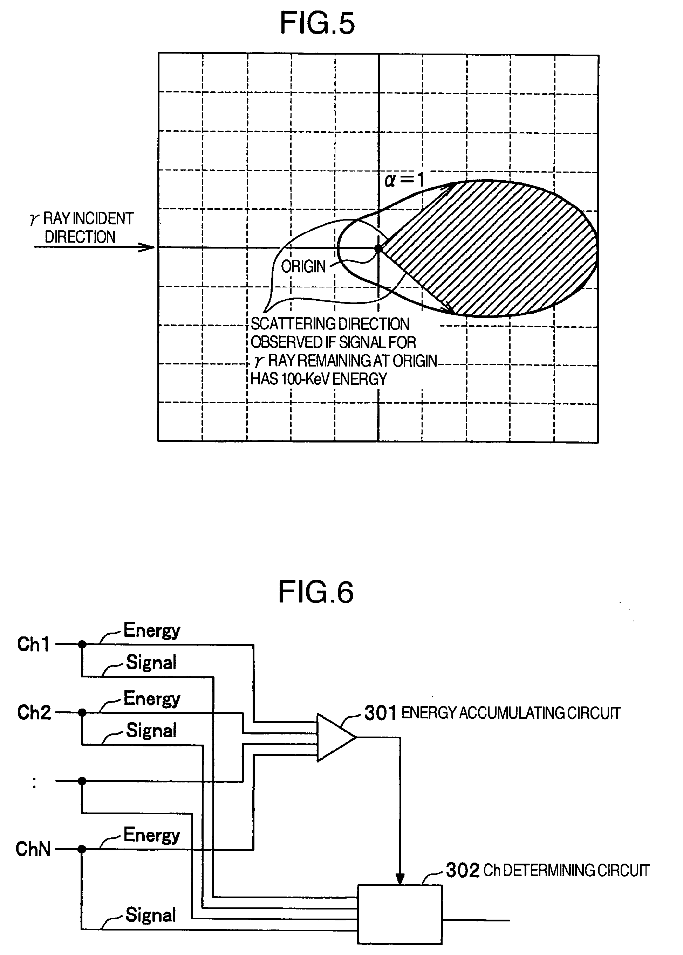

The unit data integrating device 112 couples γ ray data generated within the same unit together. It is necessary to know the direction in which γ rays are likely to scatter during the data coupling. Thus, with reference to Figure 5, description will be given of the scattering probability of a 511-keV γ ray.

Fig. 5 is a graph showing the distribution of scattering angles of a 511-keV γ ray used in PET. For 511 keV, the γ ray is likely to scatter forward. Further, the circuit used in the present embodiment cannot obtain signals of at most 100 keV under the effect of noise. In this case, in the radiological detector 4 in which a γ ray undergoes initial scattering, if the γ ray scatters in directions within the shaded range shown in Fig. 5, the signal for a γ ray remaining at the origin (radiological detector 4) has energy of at most 100 keV. Consequently, the signal for this γ ray cannot be detected. However, also for the remaining part (the entire general eclipse of α = 1 except for the shaded part), the γ ray is more likely to scatter forward.

As a result, data processing is executed on the basis of the likelihood of the γ ray scattering forward. Circuits for the data processing are integrated into the unit data integrating device 112. The data processing uses an analog or digital circuit. In the present embodiment, either of these processes is executed.

Fig. 6 is a diagram showing the configuration of a circuit for the analog processing. If the signal obtained by a radiological detector 4 exceeds a predetermined threshold, it is separated into a signal and an energy used to send a peak value. First, an energy accumulating circuit 301 sums peak values input during each time zone having a predetermined time interval and within which all the signals generated are considered to be coincident. If the sum of the peak values is within a predetermined range, the signals are considered to be processed. For a PET apparatus, when, for example, the sum of the peak values is between 400 and 550 keV, the signals are considered to be processed. If the energy accumulating circuit 301 considers the signals input during the time zone to be processed, it sends this information to a Ch (channel) determining circuit 302 as a trigger. Upon receiving the trigger from the energy accumulating circuit 301, the Ch determining circuit 302 determines the signal from one of the radiological detectors 4 having input the signals during the time zone, to be a representative value. The determining process will be described.

If only one signal is input to the energy accumulating circuit, that channel Ch is determined to be true. For example, if a signal is sent only from a channel ChN, the energy accumulating circuit sends a signal ChN. If at least two signals are input to the energy accumulating circuit, one of the signals which is closest to the center of the apparatus (the center of the radiological inspection apparatus, specifically, the center of the imaging device 2 shown in Fig. 1; this applies to the description below) is determined to be true. For example, if signals occur in channels Ch1 and Ch3, the signal for Ch1 corresponds to the stage located closest to the center of the apparatus (signal 4f shown in Fig. 1). The signal for Ch3 corresponds to the second stage from the center of the apparatus (signal 4e shown in Fig. 1). In this case, the Ch determining circuit 302 knows that Ch1 is located before Ch3 (closer to the center of the apparatus) and outputs the signal for Ch1. If there are at least two signals from the radiological detectors 4 located closest to the center of the apparatus, the energy accumulating circuit sends, for example, a signal is transmitted which is indicative of the position of center of gravity of these radiological detectors 4 (the average of positional coordinates).

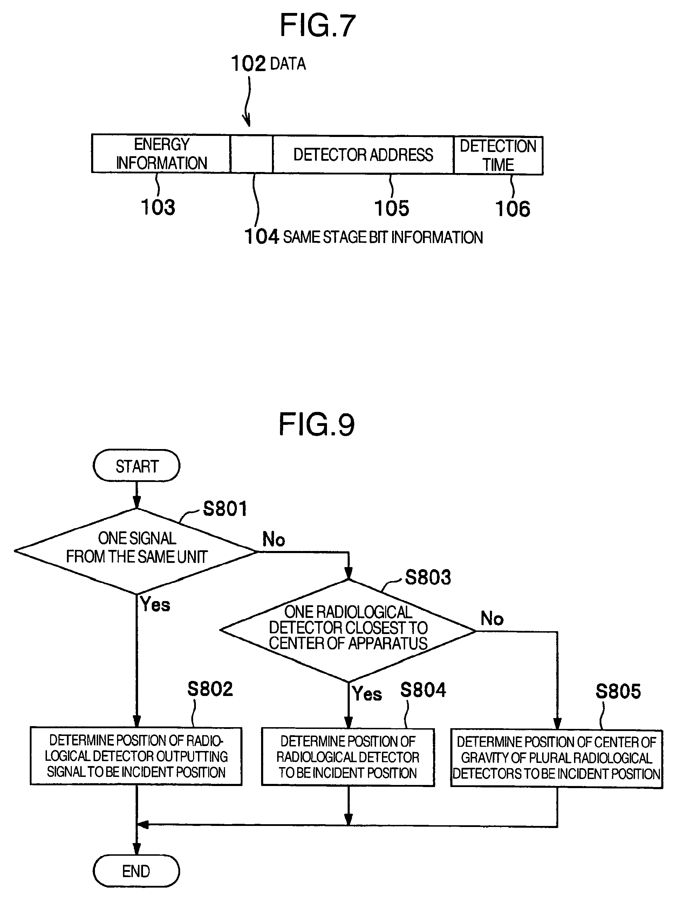

With digital processing, signals obtained by the radiological detectors 4 are sequentially sent starting with the innermost radiological detectors 4, using a data format shown in Fig. 7. Data 102 is composed of energy information 103, same stage bit information 104, a detector address 105, and a detection time 106. The energy information 103 indicates the magnitude of energy detected by each radiological detector 4. The same stage bit information 104 is a bit indicating whether or not the following data relates to the same stage (for the stages, see the description of Fig. 1 and the like). For example, if two signals are detected and contain data relating to different stages, the same stage bit information 104 is 0 in the data 102 in both signals. On the other hand, when both signals contain data relating to the same stage, the same stage bit information 104 in the first data 102 is 1. The same stage bit information 104 in the next data 102 is 0 because there is no more data. Thus, this data format deals with the case where a plurality of data 102 are detected in the same stage. The detector address 105 is a unique number assigned to each radiological detector 4. The detection time 106 is the time at which the radiological detector 4 detected a γ ray. The unit data integrating device 112 transmits all the signals obtained from the radiological detectors 4 within the same unit, to the γ ray distinguishing device 8 for each time zone having the predetermined time interval and within which all the signals generated are considered to be coincident.

With reference to Fig. 1, the unit 101 such as the unit 101c in which only the radiological detector 4d has generated a signal transmits only that signal to the γ ray distinguishing device 8 via the unit data integrating device 112. On this occasion, the same stage bit information 104 in the data 102 is 0.

On the other hand, the unit 101 such as the unit 101a in which the two radiological detectors 4e and 4f have generated signals sends the two data 102 to the γ ray distinguishing device 8 via the unit data integrating device 112. For example, in Fig. 1, the data 102 from (B) reaches the γ ray distinguishing device 8 before the data 102 from (A) does. When there are two data, the order of the data is actually unknown in terms of the devices. However, in terms of the processing, the order of the data can be determining by estimating the incident positions.

The γ ray distinguishing device 8 has a function for removing signals for scattered γ rays. Positrons emanated by the PET radiopharmaceuticals generate 511-keV γ ray energy in the body by positron annihilation. However, if the γ rays scatter in the body, the energy becomes lower than 511 keV. Further, the scattered γ rays do not pass through a position at which the γ ray pair has been generated. Accordingly, the scattered γ rays cannot be utilized to identify the position. Thus, the γ ray distinguishing device 8 executes a determining process such as the one described below. On the basis of the determination, the γ ray distinguishing device 8 removes signals or sends them to the coincidence counting device 9.

The unit 101c provides one signal containing one data 102. Accordingly, the γ ray distinguishing device determines whether or not the energy of the signal exceeds a threshold. On the other hand, the unit 101a provides two signals containing the respective data 102. Accordingly, even if the value for the energy of each signal is equal to or smaller than the threshold, the γ rays may have scattered inside the radiological detectors 4. Thus, if at least two γ rays are incident, the energy information 103 is extracted from each of the incident signals. Then, a lookup table is used to determine whether or not the γ rays have scattered inside radiological detectors 4, that is, whether or not the signals are to be processed. The lookup table is stored in predetermined storage means provided in the γ ray distinguishing device 8.

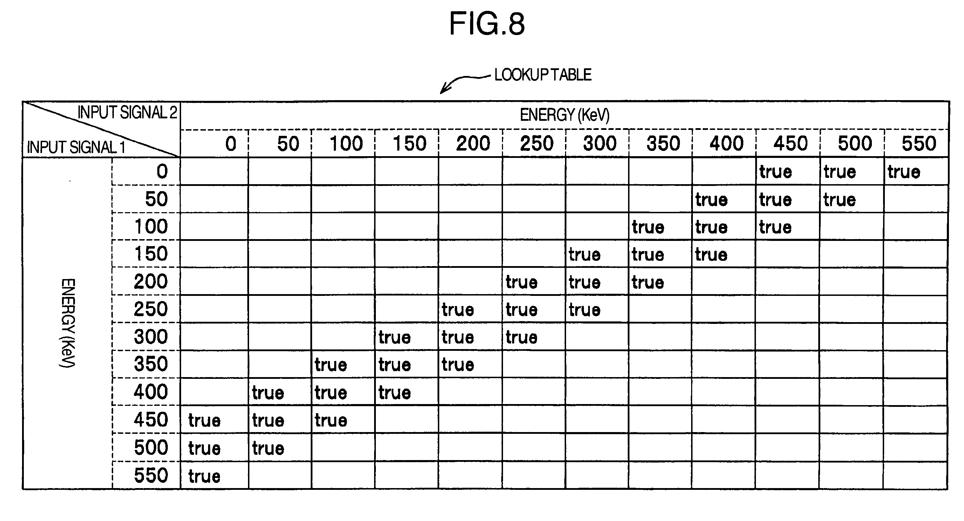

Fig. 8 is a diagram showing a lookup table corresponding to two γ rays. The lookup table indicates whether or not two input signals are to be processed ("true" in Fig. 8) depending on the combination of the energy values of the signals. The energies of the input signals 1 and 2 are shown in 50 keV increments. This indicates that, for an energy value of at least 0 keV and less than 50 keV, a row or column for 0 keV is referenced. That is, the table shows the lower limit values for the ranges of energy. In this case, 1 and 2 in the input signals 1 and 2 do not means a temporal order. For example, a signal for the data 102 with a smaller value for the detector address 105 may be defined as 1, whereas a signal for the data 102 with a larger value for the detector address 105 may be defined as 2.

By way of example, the case will be considered in which the energy information 103 in the signal (B) is 111 keV, whereas the energy information 103 in the signal (A) is 400 keV. In the lookup table, in this case, "true" is shown in the section in which the row for the input signal 1 = 100 keV crosses the column for the input signal 2 = 400 keV. Thus, these signals are to be processed. In this case, the sum of the energy information 103 is 511 keV. With the circuit in accordance with the present embodiment, forward scattering is more likely to occur than backward scattering. One of the radiological detectors 4 having output signals which is closest to the subject is estimated to correspond to an initial scattering position. Consequently, the detector address 105 in the leading data 102 is determined to be true. The corresponding signal is then sent to the coincidence counting device 9. To determine whether or not the signals are to be processed, it is possible to sum the energy values and to check whether or not the sum is within a predetermined range as in the case of the analog processing.

The coincidence counting device 9 uses the detection time 106 in the data 102 to determine the coincidence of the signals collected for each unit. The signals determined to be coincident are transmitted to the computer 11. The data in the sent signals is positional information on the two radiological detectors 4. However, other data may be added to the positional information as required.

In this case, for example, when (A) and (B) in Fig. 1 are on the same stage, detected position information is the middle point between these two points.

The computer 11 reconstructs the data thus obtained and causes the display device 18 to display the reconstructed data.

Description has been given of the analog and digital processing executed by the unit data integrating device 112. Now, with reference to the flowchart in Fig. 9, description will be given of a basic process for estimating a γ ray incident position which is common to both analog and digital processing. The signals shown in the flowchart are input during the time zone within which all of them are considered to be coincident. The sum of the values for the signals is within a predetermined range. The signals are determined to be processed.

First, the unit data integrating device 112 checks whether or not one signal has been received from the same unit 101 (step S801). If one signal has been received (Yes in step S801), the position of the radiological detector 4 having output that signal is determined to be the γ ray incident position (step S802). If more than one signals have been received from the same unit 112 (No in step S801), the unit data integrating device 112 checks whether or not only one of the plural radiological detectors 4 having output the signals is closest to the center of the apparatus (step S803). If only one of the plural radiological detectors 4 is closest to the center of the apparatus (Yes in step S803), the position of that radiological detector 4 is determined to be the γ ray incident position (step S804). If more than one of the plural radiological detectors 4 are closest to the center of the apparatus (Yes in step S803), the position of center of gravity of the plural radiological detectors 4 is determined to be the γ ray incident position (step S805). This process enables the γ ray incident position to be appropriately determined even of a plurality of signals are received from the unit 101.

As described above, γ rays scattered inside the radiological detectors 4 can be precisely detected. This makes it possible to improve data collection efficiency without degrading the reliability of data. Further, the circuit can be simplified and have a reduced scale. This enables a reduction in the size of the apparatus and in the quantity of heat generated, thus reducing power consumption. An expensive processing device is unnecessary which has a time resolution of, for example, 0.1 nsec. Moreover, data collection efficiency is improved to enable a reduction in the time required to measure γ rays from the subject. This makes it possible to reduce the exposure of the subject to radiation.

### <Other embodiments>

An example of the preferred embodiment of the present invention has been shown. However, the present invention is not limited to the above embodiment, which may be appropriately altered without departing from the spirit of the present invention. For example, the embodiments described below are possible.
(1) In the description of the above embodiment, the unit data integrating device 112 identifies the position inside the radiological detectors 4 where a γ ray was first incident. However, for example, each radiological detector 4 may save the amount of energy attenuated and the time of the attenuation (scattering) as data so that the computer 11 can use the data to identify the γ ray incident position. This eliminates need for the γ ray distinguishing device 8 and coincidence counting device 9. Therefore, the signal processing device 7 can be simplified.
(2) In the description of the above embodiment, the unit data integrating device 112 processes the plural signals from the radiological detectors 4 within one unit 101. However, if γ rays may scatter over plural units 101, signals from the radiological detectors 4 belonging to the plural units 101 may be input to the unit data integrating device 112 via the wire 13 for processing.
(3) In the above embodiment, the PET apparatus is shown as an example of the radiological inspection apparatus 1. The same effects can be exerted using a SPECT apparatus. Further, an X ray CT apparatus can be used to similarly identify an X ray incident position.
(4) When γ rays from the subject are incident on particular radiological detectors 4 and scatter, the attenuated γ rays are incident on other radiological detectors 4. Consequently, radiological detectors 4 closer to the subject have higher detection sensitivities. Radiological detectors 4 further from the subject have lower detection sensitivities. Thus, the length of the radiological detectors 4 closer to the subject may be reduced in a direction normal to the subject's body axis (γ ray incident direction). The length of the radiological detectors 4 further from the subject may be increased in a direction normal to the subject's body axis (γ ray incident direction). This averages the number of times the radiological detector 4 detects a γ ray per unit time. Therefore, detection errors can be reduced.

It should be further understood by those skilled in the art that although the foregoing description has been made on embodiments of the invention, the invention is not limited thereto and various changes and modifications may be made without departing from the spirit of the invention and the scope of the appended claims.

## Claims

1. A radiological inspection apparatus comprising radiological detectors (4) at least installed in a plurality of stages along a direction normal to a central axis of the radiological inspection apparatus (1) to detect radiation from a testing object (17) located in a center of the detectors (4) or a vicinity of the center,
wherein if a plurality of radiological detectors (4) detect radiation, an order in which the radiation is scattered is estimated using a positional relationship between the plurality of radiological detectors (4) and a central axis of the radiological inspection apparatus (1).

2. The radiological inspection apparatus according to claim 1, wherein in estimating the scattering order, one of the plurality of radiological detectors (4) which is closest to the central axis of the radiological inspection apparatus (1) is estimated as an initial scattering position.

3. The radiological inspection apparatus according to claim 1, wherein the radiological detectors (4) are further arranged like a ring around the central axis of the radiological inspection apparatus (1).

4. The radiological inspection apparatus according to claim 1, wherein a plurality of the radiological detectors (4) are further installed in a direction of the central axis of the radiological inspection apparatus (1).

5. The radiological inspection apparatus according to claim 1, wherein the radiological detectors (4) have different lengths in the normal direction depending on the stage in which the radiological detectors (4) are installed along the normal direction.

6. The radiological inspection apparatus according to claim 5, wherein a length of the radiological detector (4) in the normal direction increases as a distance between the radiological detector (4) and the testing object (17) increases.

7. The radiological inspection apparatus according to claim 1 wherein the radiation is a γ ray, and
the radiological inspection apparatus (1) is a SPECT apparatus or a PET apparatus.

8. The radiological inspection apparatus according to claim 1 wherein the radiation is an X ray, and
the radiological inspection apparatus (1) is an X-ray CT apparatus.

9. A radiological inspection method for a radiological inspection apparatus (1) comprising radiological detectors (4) at least installed in a plurality of stages along a direction normal to a central axis of the radiological inspection apparatus (1) to detect radiation from a testing object 17 located in a center of the detectors (4) or a vicinity of the center,
wherein if a plurality of radiological detectors (4) detect radiation, an order in which the radiation is scattered is estimated using a positional relationship between the plurality of radiological detectors (4) and a central axis of the radiological inspection apparatus (1).

10. The radiological inspection method according to claim 9, wherein in estimating the scattering order, one of the plurality of radiological detectors (4) which is closest to the central axis of the radiological inspection apparatus (1) is estimated as an initial scattering position.

11. The radiological inspection method according to claim 9, wherein the radiological detectors (4) are further arranged like a ring around the central axis of the radiological inspection apparatus (1).

12. The radiological inspection method according to claim 9 wherein the radiation is a γ ray, and
the radiological inspection apparatus 1 is a SPECT apparatus or a PET apparatus.

13. The radiological inspection method according to claim 9 wherein the radiation is an X ray, and
the radiological inspection apparatus (1) is an X-ray CT apparatus.
